(19) Europäisches Patentamt
European Patent Office
Office européen des brevets

(11) Publication number: **0 351 418 B1**

# (12) EUROPEAN PATENT SPECIFICATION

(45) Date of publication of patent specification: **17.05.95** (51) Int. Cl.$^6$: **C12N 15/86**

(21) Application number: **88903647.1**

(22) Date of filing: **21.03.88**

(86) International application number:
**PCT/US88/00986**

(87) International publication number:
**WO 88/07088 (22.09.88 88/21)**

## (54) VIRAL VECTOR SYSTEM FOR THE INTRODUCTION OF FOREIGN DNA EXPRESSION PRODUCTS INTO GALLINACEOUS BIRDS.

(30) Priority: **19.03.87 US 27724**

(43) Date of publication of application:
**24.01.90 Bulletin 90/04**

(45) Publication of the grant of the patent:
**17.05.95 Bulletin 95/20**

(84) Designated Contracting States:
**AT BE CH DE FR GB IT LI LU NL SE**

(56) References cited:
**WO-A-87/00862**
**WO-A-87/04463**
**WO-A-89/01040**

**Virology, Volume 157, issued April 1987 (New York, New York), (IGARASHI et al.), "Restriction Enzyme Map of Herpesvirus of Turkey DNA and its Colinear Relationship with Marek's Disease Virus DNA", see page 351**

(73) Proprietor: **SYNERGEN, INC.**
**1885 33rd Street**
**Boulder**
**Colorado 80301 (US)**

(72) Inventor: **MARTIN, Sandra**
**1608 Daphne Street**
**Boulder, CO 80020 (US)**
Inventor: **BANDYOPADHYAY, Pradip**
**1645 14th Street 406**
**Boulder, CO 80302 (US)**

(74) Representative: **Patentanwälte Grünecker, Kinkeldey, Stockmair, Aufenanger & Partner**
**Maximilianstrasse 58**
**D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 351 418 B1

Journal of Virology, Volume 53, issued March 1985, (Washington, D.C., U.S.A.),(FUKUCHI et al.), "Map Location of Homologous Regions Between Marek's Disease Virus and Herpesvirus of Turkey and the Absence of Detectable Homology in the Putative Tumor-Inducing Gene", see pages 994 and 995

Journal of General Virology, Volume 64, issued April 1983, (London, England),(IKUTA et al.), "Most Virus-Specific Polypeptides in Cells Productively Infected with Marek's Disease Virus or Herpesvirus of Turkeys Possess Cross-Reactive Determinants", see page 961

Virology, Volume 161, issued December 1987, (New York, New York) (MAVROMARA-NAZOS et al.), "Activation of Herpes Simplex Virus 1 gamma2 Genes by Viral DNA Replication", see page 593

Science, Volume 236, issued 1 May 1987, (Washington, D.C., U.S.A.), (WEBER et al.), "Rapid Identification of Nonessential Genes of Herpes Simplex Virus Type1 by TN5 Mutagenesis", see page 576

Science, Volume 236, issued 1 May 1987, (Washington, D.C., U.S.A.), (LONGNECKER et al.), "Clustering of Genes Dispensable for Growth in Culture in the S Component of the HSV-1 Genome", see page 573

Proceedings of the National Academy of Sciences, Volume 81, issued June 1984,(Washington, D.C., U.S.A), (GIBBS et al.), "Extensive Homology Exists Between Marek Disease Herpesvirus and its Vaccine Virus, Herpesvirus of Turkeys" seepage 3365

Proceedings of the National Academy of Sciences, Volume 79, issued January 1982, (Washington, D.C., U.S.A.), (CEBRIAN et al.), "Inverted Repeated Nucleotide Sequences in the Genomes of Marek Disease Virus and the Herpesvirus of the Turkey", see page 555

Science, Volume 229, issued 30 September 1985, (Washington, D.C., U.S.A.), (ROIZMAN et al.), "Genetic Engineering of Novel Genomes of Large DNA Viruses", see pages 1208 and 1212-1213

The Journal of General Virology, Volume 64, issued December 1983, (London,England), (KAZUYOSHI et al.), "Monoclonal Antibodies Reactive with the Surface Glycoproteins of Marek's Disease Virus and Herpesvirus of Turkeys" see page 2597

Glycoproteins of Marek's Disease Virus and Herpesvirus of Turkeys", see page 2597

## Description

### BACKGROUND OF THE INVENTION

The present invention relates to a method for introducing a gene of interest into a gallinaceous bird and a method for the creation of a viral vector capable of introducing a functional gene of interest into a gallinaceous bird. The invention relates further to a vector for expressing a gene of interest in a gallinaceous bird. Specifically, this invention relates to the use of the herpesvirus of turkeys (HVT) to deliver and express foreign genes into gallinaceous birds and to induce immunity against Marek's disease.

The herpesvirus of turkeys, particularly HVT strain FC126, is currently used in the poultry industry in vaccines to protect against the oncogenic Marek's disease virus (MDV). Virtually all broiler chicks are inoculated with HVT on day 1 after hatching. HVT is a naturally-occurring field virus isolated from turkeys and is not oncogenic for either chickens or turkeys. HVT normally induces viremia in chickens, and the response peaks between 8-15 days after inoculation. Although there is evidence that a gradual decrease in the titer of HVT from inoculated birds occurs over time, 58% of the chickens in one study still had recoverable levels of HVT 76 weeks after vaccination as reported by Phillips and Biggs, J. Natl. Cancer Inst. 49:1367-1373, 1972.

The persistence of this virus in the chicken makes it highly probable that products of exogenous genes which could be expressed during the infection would also be available to the host for a long period, possibly through harvest time for broilers. Thus, two reasons support the use of HVT as a viral vector. First, HVT is already widely used throughout the industry to protect against Marek's disease. Second, HVT provides a sound means for achieving a continuous supply of a non-viral gene product in poultry.

WO 87/04463 discloses a method involving administration of hybrid non-primate herpes virus to an animal host, wherein said virus includes a foreign DNA sequence adapted to expression in said host. In example 15, construction of a hybrid HVT by insertion of the $\beta$-gal gene under control of the PRV-gpX promoter into the XhoI site in BamHI#16 (unique long region of HVT is disclosed.

Roizman & Jenkins, (science, Vol.229, pp. 1208-1214 (1985)) provide a review article concerning methods of genetic engineering of large DNA viruses, disclosing inter alia the general genomic organisation of the HSV-1 genome (Fig. 1) and certain sites, which have been shown to be non-essential for growth and cell culture, including "various portions of the domain of the TK gene".

Cebrian et al. (PNAS, Vol. 79, pp.555-558 (1982)) disclose a comparison between Marek disease virus and turkey herpes virus, which are biologically similar to herpes viruses of the group comprising Epstein-Barr virus, H. saimiri, H. ateles. However, with respect to the genomic structure, Marek disease virus and turkey herpes virus were shown to have inverted repeats and thus to resemble herpes viruses of the sub-family Alpha-herpesvirinae, said sub-family including herpes simplex virus (HSV).

### SUMMARY OF THE INVENTION

It is an object of the present invention to provide viral vectors capable of functioning as vaccines against Marek's disease which are also capable of introducing a functional gene of interest (GOI) into a gallinaceous bird. It is also an object of the present invention to provide methods for the construction or these viral vectors.

Additional objects and advantages of the present invention will be set forth in part in the description which follows, or may be learned from practice of the invention. The objects and advantages may be realized and attained by means of the instrumentalities and combinations particularly pointed out in the appended claims.

To achieve the objects and in accordance with the purposes of the present invention, a method for introducing a gene of interest into a gallinaceous bird is disclosed. This method comprises inserting the gene of interest into a site within a non-essential region of herpes virus of turkeys (HVT) which is non-oncogenic in gallinaceous birds and is capable of being used as a vaccine against Marek's disease.

Thus created, the viral vector is used to infect a gallinaceous bird, causing the gene of interest to be expressed.

Two methods, the Backwards and Forwards methods, for creating these viral vectors are disclosed. In the Forwards method, a viral vector capable of introducing a functional gene of interest into a gallinaceous bird is created by:

a. Locating a site within a non-essential region in the genome of a herpes virus of turkeys (HVT), wherein the non-essential site is located within the 6.25 kb Sal I fragment depicted in Fig 1;

b. Inserting the gene of interest into said site within the non-essential region of the viral genome to create a viral vector.

The Backwards method for the creation of a viral vector capable of introducing a functional gene of interest into a gallinaceous bird comprises:

a. Identifying a viable herpes virus of turkey (HVT) which has a genomic defect which is manifested in an identifiable phenotype; and

b. Inserting the gene of interest coupled to a DNA sequence capable of repairing the viral defect into a site within the 6.25 kb Sal I fragment of the virus depicted in Fig. 1 to create a viral vector. It is understood that the foregoing general description in the following Detailed Description are exemplary and explanatory only and are not restrictive of the invention as claimed. The accompanying drawings, which are incorporated in and constitute a part of this specification, illustrate one embodiment of the invention, and, together with the description, serve to explain the principles of the invention.

## BRIEF DESCRIPTION OF THE DRAWINGS

Figure 1. Physical Map of the Region of DNA Encoding HVT TK. The location of SalI restriction endonuclease cleavage sites for EMBL3 phage clone E11 are indicated. Fragments are drawn approximately to scale, where the total length of the insert is about 21 kilobase pairs. The two end sites are from the vector, not from the HVT DNA insert. The 6.25 kb Sal I fragment is expanded to show the location of the kan insertions that have been recovered in viral DNA. Insertions of sites 1-4 can be recovered following selection of HVT that grows in the presence of AraT (forward selection). Insertions at sites 5-9 are found following rescue of the AraT° growth defect (Backwards selection). The exact locations of sites 1-6 are known from DNA sequence data, locations of sites 7-9 are from restriction mapping. Sites are as follows: 1. HpaI, 2. XbaI, 3. SspI, 4. SspI, 5. SspI, 6. SspI, 7. ClaI, 8. NsiI, 9 SnaBI. The region for which DNA sequence is presented (Figure 3) is indicated, and the expanded region shows the location of the two open reading frames. ORF 1 is believed to be the HVT thymidine kinase locus, also referred to in this application as locus X.

Figure 2. The region of 11A that carries AraT resistance is probably TK. The 6.25 kb SalI fragment from 11A is shown, with an asymmetric EcoRI site that divides it into 2.0 and 4.25 kb segments. Indicated at the top is the segment of DNA that is required to restore wild-type phenotype to ATR°. Below are shown the endpoints of the various EMBL3 clones that contain all or part of 11A and the region of DNA that remains in a series of deletion mutants we have made in the original 11A subclone.

Figure 3. DNA sequence of 2,720 base pairs in the region encoding HVT TK. Clones of both the large and small EcoRI-SalI subfragments were made in bacteriophage M13. In the small fragment, 2,026 base pairs, the DNA sequence from both strands was determined by cloning the fragment in both orientations in M13mp18 then creating a series of deletions from the sequencing primer site using the method of Henikoff (Gene 28, 351-359, 1984). 3' of the EcoRI site (Figure 1) DNA sequence has been generated using a series of primers to walk along a M13mp19 subclone of hte 4.25 kb EcoRI-SalI subfragment described in Figure 2. Sequence has been determined using the dideoxy method (Sanger et al., J. Mol. Biol. 143, 161-178, 1980).

## DETAILED DESCRIPTION OF THE PREFERRED EMBODIMENT

Reference will now be made to the preferred embodiments of the present invention. As noted above, the invention relates to the use of the described viral vectors in all gallinaceous birds. However, for the purposes of this discussion, chickens will be referred to as exemplary of gallinaceous birds. In addition, while HVT is described in detail herein as the presently preferred embodiment of the invention, use of other viruses as vectors are also contemplated. These other viruses include viruses (1) which are not oncogenic in the gallinaceous bird host; (2) which contain at least one "nonessential" site into which foreign DNA can be inserted; and (3) which can be used as a vaccine for Marek's disease. Thus, the viruses encompassed within this invention include, but are not limited to HVT and any other virus which can protect against Marek's disease, such as Marek's disease virus (MDV) serotype 2 or MDV serotype 1, attenuated.

Prior to a detailed discussion of this invention, definitions for certain terms used in the discussion should be established. "Nonessential" is intended to refer to a portion of the viral genome which is not necessary for virus propagation. By "foreign DNA," it is intended to mean DNA which codes for a gene not naturally found in the viral vector.

HVT contains a DNA genome of about 180 kilobase pairs (180 kbp) and has the potential for carrying a number of recombinant genes for delivery. Generally in order to express foreign genes in a viral vector and to ensure that the virus continues to grow, the foreign genes must be inserted into regions of the viral genome that are nonessential for viral growth. The locus X, indicated on the map of a portion of the the HVT viral genome in Figure 1, was chosen as such a region because it was found to be nonessential for virus growth. While the present inventors believe that locus X is the thymidine-kinase locus, this has not yet been conclusively proven. However, for purposes of the discussion herein, the locus X depicted in Fig. 1 will be referred to as the thymidine-kinase (TK) locus.

Other possibilities include some of the various glycoprotein genes, believed to be nonessential because analogous proteins were found to be nonessential in the herpes simplex and pseudorabies viruses and especially the HVT protein A which is thought to be nonessential for growth and for protection against Marek's Disease. In addition, the thymidine-kinase locus (TK), if it is different from locus X, would also be a useful insertion site because TK has been shown to be nonessential in other large DNA viruses, including herpes simplex, pseudorabies and vaccinia viruses.

Various foreign DNA molecules, also referred to herein as "genes of interest" or "GOI," are contemplated for insertion into the viral vector. For example, antigens may be inserted into the vector and used to induce immunity to other fowl diseases. These antigens include, but are not limited to, antigens from other viruses, coccidia antigens and paramyosin, a muscle protein common to all invertebrate parasites, particularly shistosomes. In addition, analogs of somatostatin have been proposed for use in order to increase the fowl growth rate. Moreover, hormones such as growth promoters and immune regulators capable of growth stimulation or disease protection may be used.

As says for in vivo and in vitro expression of the genes of interest are contemplated. These include radioimmunoassays (RIAs) or enzyme linked immunosorbent assays (ELISAs) used for detecting foreign protein in cultured cells as well as in chicken serum. Also included are tests for in vivo IgA and IgG antibody levels to foreign proteins.

Two general schemes are contemplated for the creation of the viral vectors of the present invention. The first, designated herein as the "forward" scheme, involves the insertion of a GOI into a nonessential region of the viral genome. In a preferred embodiment of the present invention, this method is used to insert a GOI into the TK region of the genome. This results in a viral vector which exhibits an AraT resistant phenotype.

In a second method of practicing the present invention, hereinafter referred to as the "backwards" method, a virus which is viable but which has a genomic defect which is manifested in an identifiable manner is used. The GOI which is inserted into this defective or "crippled" virus is coupled with a DNA sequence capable of repairing the viral defect. For example, in a virus which has a defective TK region, and thereby exhibits AraT-resistance, the GOI is coupled with a DNA sequence capable of restoring TK. Thus, restoration of TK, resulting in AraT sensitivity, is indicative of the insertion of the GOI into the viral vector. It should be noted that viruses with defects in the TK gene generally grow more slowly than the wild-type virus. Thus, successful restoration of TK will result in a viral vector which has a growth advantage over the mutant strain.

Although HVT has been widely used as a vaccine for Marek's disease since 1972, prior to the present invention HVT was virtually uncharacterized at the molecular and genetic levels. Thus, many tools and techniques had to be developed as prerequisites for the production of a recombinant HVT which could express a foreign gene. These included propagation of HVT, preparation of viral DNA, preparation of cloned DNA libraries of the HVT genome, preparation of infectious viral DNA for transfections, and development of a selection tool to use in identifying the desired viral recombinants. Success in each of these areas has enabled the inventors to define regions in the HVT viral DNA where the insertion of foreign DNA will not jeopardize the original function of HVT. The methods and means used to develop each of these prerequisites are described briefly below. These methods and means may be readily adapted, through use of the processes known to those of ordinary skill in the art applied in light of the teachings contained herein, to develop the tools necessary to develop the other viral vectors encompassed within the scope of the present invention.

The following examples are designed to elucidate the teachings of the present invention and not to limit its scope. Various other modifications and equivalents of the examples will readily suggest themselves to those of ordinary skill in the art, particularly after issuance of this patent, without departing from the scope or spirit of the invention.

## EXAMPLES

## EXAMPLE 1

### MOLECULAR AND GENETIC CHARACTERIZATION OF HVT AND DEFINITION OF TK REGION

Primary cultures of chicken embryo fibroblasts (CEF) were prepared according to procedures described in Culture of Animal Cells by Freshney, R.I. (Alan R. Liss Inc., New York, 1983), specifically incorporated herein by reference, and were grown in medium M199 supplemented with bovine fetal serum. The cells were then infected with herpesvirus of turkeys (HVT) strain FC126 which is used in the industry to vaccinate poultry against Marek's disease. FC126 can be purchased from Salsbury Labs, Charles City, Iowa. When the cells exhibited approximately 70% cytopathy, they were harvested and high molecular weight DNA was isolated according to procedures described by Maniatis et al. in Molecular Cloning (Cold Spring Harbor, New York), specifically incorporated herein by reference. HVT DNA represented about one to ten percent of total infected cell DNA.

This HVT DNA was partially digested with restriction endonuclease Sau3AI, and DNA fragments ranging from 15-20 X $10^3$ base pairs in length were isolated according to the procedures described by Maniatis et al., supra. The size selected DNA fragments were ligated to bacteriophage lambda vector EMBL3 which had previously been digested with the restriction endonuclease BamHI. The ligated DNA was packaged in vitro using commercially available packaging extracts obtained from Vector Cloning Systems (San Diego, California) according to the procedures described by the vendor.

Packaged DNA was used to infect bacterial strain Q359 which was then plated on LB agar. DNA obtained from the resulting plaques was transferred to nitrocellulose filters and hybridized to $^{32}P$ labeled HVT DNA. ($^{32}P$ labeled HVT DNA was obtained by nick-translation of electrophoretically purified HVT DNA in the presence of alpha $^{32}P$dCTP according to procedures described by Maniatis et al., supra. Hybridization positive plaques were picked. They constitute the HVT recombinant DNA library used in the subsequent experiments. The HVT recombinant DNA library consists of overlapping sequences of HVT DNA in bacteriophage lambda.

High molecular weight DNA isolated from CEF infected with HVT was used to transfect uninfected CEF using the calcium phosphate precipitation technique described by Graham, F.L. and Van der Eb, A.J., Virology 52:456-467 (1973), specifically incorporated herein by reference. Infectious virus was recovered four to five days after transfection.

1. Identification of the HVT TK Gene

Insertion of foreign DNA into the HVT genome depends upon the relatively rare event of homologous recombination between infectious viral DNA and a plasmid carrying the gene of interest embedded within HVT sequences following co-transfection of the two DNAs. Thus, some method had to be developed that would allow one to distinguish between the input infectious DNA and the desired recombinants in the inevitable mixed population of plaques resulting from co-transfection.

2. Locus X AraT Resistant Variant of $TK^-$

a. Selection of an AraT Resistant Variant of HVT.

It was demonstrated that CEF infected with HVT incorporated significantly more $^{125}I$-deoxycytidine-($^{125}IdC$) into DNA than mock-infected cells (Table 1). In HSV-infected cells, $^{125}IdC$ is incorporated into DNA only after it has been phosphorylated by the viral TK as described by Summers and Summers, J. Virol. 24: 314-318 (1977), specifically incorporated herein by reference. Moreover, growth of HVT in CEF is inhibited by the drug AraT, whereas uninfected CEF appears to be unaffected. AraT (1-beta-D-arabinofuranosylthymine obtained from Calbiochem) is lethal to cells only once it is incorporated into the DNA where it acts as a chain-terminator. Again by analogy to HSV, AraT-resistance is likely to be due to mutations in the thymidine kinase gene.

TABLE 1

| | MOI | cpm/mg protein |
|---|---|---|
| CEF, uninfected | n.a. | 25,400 |
| + HVT | .001 | 37,500 |
| + HVT | .01 | 73,700 |
| + HVT | .02 | 103,200 |
| + HVT | .05 | 175,400 |
| + HVT | .1 | 202,000 |
| + HVT | .2 | 202,500 |
| + HVT | .5 | 291,900 |
| Vero, uninfected* | n.a. | 287,900 |
| + HSV-1 | .01 | 1,625,800 |
| + HSV-1 | .1 | 6,108,200 |
| + HSV-1 | .5 | 7,093,200 |

*vero cells infected with HSV-1 are included for comparison.

Twelve-well plates were inoculated with cells 3 hours prior to infection. Cells were infected at varying multiplicities of infection (MOI). $^{125}$I-iododeoxycytidine was added to the medium 2 hours post-infection at concentrations of 5 x $10^5$ cpm/ml. The labeling period was 16 hours.

Cells were harvested by precipitation on plates with cold 5% TCA (trichloroacetic acid). Wells were washed 3 times with 5% TCA. Cell material was hydrolyzed in 0.5N NaOH, then aliquots were taken for counting and protein determination.

The sensitivity of wild-type HVT to AraT suggested that it may be feasible to identify the TK locus in HVT by selecting an AraT-resistant variant.

3. Isolation of AraT Resistant HVt

AraT-resistant variants occur spontaneously in populations of FC126, and are isolated by growing cell-free virus as described by Calnek et al., Avian Dis. 16:52-56 (1972), specifically incorporated herein by reference, in CEF in the presence of 100 ug/ml AraT. Specifically, 150 mm tissue culture dishes are seeded with 2 X $10^7$ cells of secondary CEF. Two hours later the medium is removed, and 2 X $10^4$ pfu of cell-free HVT are adsorbed for 30 minutes. After adsorption, 30 ml of medium containing 100 ug/ml of AraT is added. Cells are fluid-changed every two days, always with AraT, and monolayers are screened for plaques 4-5 days post-infection at 40-fold magnfication using an inverted microscope. One AraT-resistant variant ATR° was found in 2 X $10^5$ pfu screened initially. Subsequent experiments have yielded AraT resistant HVT at least that frequency. ATR° remains stable for at least nine passages in the absence of AraT.

TABLE 2

| Incorporation of $^{125}$I-deoxycytidine ($^{125}$IdC) in CEF cells infected with wild-type or ATR° HVT | |
|---|---|
| | cpm/mg Protein |
| Mock-infected | 8,622 |
| Wild-type HVT | 42,833 |
| ATR° HVT | 8,807 |

ATR° also fails to incorporate $^{125}$IdC into DNA (Table 2), as is expected for a $TK^-$ virus.

The ability of ATR° to grow in culture in the presence of FMAU (1-(2-fluoro-2-beta-D-arabinofuranoysl)-5-methyl-uracil) has been studied. This compound is commonly used as an anti-herpesvirus agent and is known to be specific for viral TK in the case of HSV. A $TK^-$ recombinant was expected to grow in the presence of this drug while the drug would inhibit the growth of wild-type HVT. ATR° grows in FMAU, but less well than wild-type HVT in the absence of drug (Table 3). ATR° also grows more slowly than the wild-type virus in the absence of either AraT or FMAU. The reduced growth rate and virus yield of ATR° made it crucial to determine the ability of ART° to protect chickens against Marek's disease. A limited animal experiment was performed, the results from which are shown in Table 4. These results show that ATR° protects chickens against Marek's disease. Thus, it was deemed worthwhile to pursue the region of DNA that confers AraT resistance (Locus X, probably TK) as a site at which to insert foreign DNA.

TABLE 3

| Assay of wild-type HVT and ATR° on chick kidney cells* | | |
|---|---|---|
| Virus | FMAU | Foci |
| WT | 0 | + |
| WT | 1uM | - |
| ATR° | 0 | + |
| ATR° | 1uM | + |

*Cell-free virus was inoculated onto chicken kidney cells in the presence or absence of FMAU. Agar overlays were applied one day post-infection, and plaques were scored 5 days post-infection.

**TABLE 4**

Assay of ATR° in chickens for protection against Marek's disease

| Vaccine[1] | Number of Birds with Marek's Disease 9 Weeks Post Challenge | | | | | | |
|---|---|---|---|---|---|---|---|
| | Challenge[2] | # of Birds | HVT Viremia | Dead | Survivors with lesions | Total Marek's | % |
| None | - | 19 | n.d.[3] | 0 | 0 | 0 | (0) |
| Wild-type | - | 19 | 18/19 | 0 | 0 | 0 | (0) |
| ATR° | - | 20 | 0/20 | 0 | 0 | 0 | (0) |
| None | + | 19 | n.d. | 15 | 1 | 16 | (84) |
| Wild-type | + | 17 | n.d. | 1 | 0 | 1 | (6)[4] |
| ATR° | + | 18 | n.d. | 1 | 3 | 4 | (22)[4] |

[1] Genetically susceptible, pathogen-free, P2 chickens were vaccinated by subcutaneous injection of infected CEF. The wild-type (strain FC126) inoculum was 238 pfu/dose, and the ATR° inoculum was 3,400 pfu/dose.

[2] 10 days post-vaccination, challenge chicks were moved to a separate isolation unit and received an intraabdominal injection of 500 pfu of cell-associated JM-10 strain (virulent Marek's disease) virus.

[3] Not done

[4] Significant protection ($p$ 0.001, chi square test)

Several additional AraT-resistant mutants of HVT were subsequently isolated. Three of these that have been tested also failed to incorporate $^{125}$IdC into DNA. These AraT-resistant mutants, like ATR°, seem to grow more slowly than wild-type HVT. The plaques are small and they consistently give lower titers.

4. Identification of the DNA Region Encoding the AraT-Resistance Phenotype

All of the AraT resistant mutants are characterized by their ability to form plaques in the presence of AraT (which completely inhibits the plaque-forming ability of wild-type HVT) and by their inability to incorporate $^{125}$I-deoxycytidine ($^{125}$IdC) into DNA. In addition, wild-type HVT has a growth advantage over the AraT-resistant variants. These differences between the wild-type HVT and HVT that are AraT-resistant, form the basis for a marker-rescue assay that allows for the identification of the region of DNA, and thus the gene which confers resistance to AraT. DNA from the AraT resistant HVT (10 ug of total infected-cell DNA) is co-transfected with individual DNA clones (200 ng of plasmid DNA or 1 ug of phage DNA) representing the genome of HVT. Two days after the DNA transfection, cells are switched from medium with 5% serum to medium with 2% serum. On day five, the transfected cells are split from their 60 mm dish to a 100 mm dish and 1 X $10^6$ fresh secondary CEF are added. On day 8, monolayers are screened under the microscope for plaques and, when present, cell-free virus is made. 0.2 ml of the cell-free virus is adsorbed to fresh monolayers of CEF on 60 mm dishes. 40-45 hours post-infection the medium is replaced with 2 ml of 2% serum in M199 (Gibco) containing 1.36 uCi/ml of $^{125}$IdC (Amersham). After 24 hours in the presence of $^{125}$IdC, cells and plaques are fixed in 50% methanol and stained with 0.4% crystal violet in 50% methanol. Plates are dried then autoradiographed. DNA from the clones containing the wild-type version of the AraT resistant region recombine with the infectious AraT$^R$ viral DNA and the resulting viruses are restored to wild-type growth, including the ability to incorporate $^{125}$IdC into DNA, and a sensitivity to AraT.

The results of this assay as set forth in Table 5, unambiguously identified a 6.3kb SalI fragment from EMBL3 clone 11 as the piece of DNA which was altered in the AraT resistant mutants. Deletions in this piece of DNA have further characterized the location of the mutation to a 1000 base pair region of DNA within this fragment (Figures 2).

TABLE 5

| Marker rescue of ATR° by various DNAs $^{125}$IdC Incorporation into Plaques | | |
|---|---|---|
| EMBL3 Clones:[1] | 117 | - |
| | 119 | - |
| | 20 | - |
| | 118 | - |
| | 121 | - |
| | 113 | - |
| | 11 | + |
| | 13 | |
| | 108 | - |
| | 112 | + |
| | 114 | + |
| | 116 | + |
| | 123 | - |
| | 53 | - |
| | 106 | - |
| | 105 | - |
| | 110 | - |
| Plasmids: | 11A | + |
| | delE1[2] | + |
| | delH10 | + |
| | delN9 | + |
| | delN3 | + |
| | delC14 | + |
| | delS7 | + |
| | delNH9 | + |
| | delR2 | - |

[1]These clones represent mapped HVT DNA. The positive phage clones share a region DNA as shown in Figure 2.
[2]All of these plasmids contain deletions in 11A as indicated on the map in Figure 2.

5. The DNA Sequence

The DNA sequence of 2720 nucleotides has been determined using standard dideoxy sequencing methods (Figure 3). The known sequence encompasses the 2 Kb SalI to EcoRI fragment (Figure 1) and continues an additional 69 mucleotides to the right of the EcoRI site. Analysis of this sequence using programs purchased from International Biotechnologies, Inc. reveal two long open reading frames, the right-most of which (ORF 1, Figure 1) shows conservation between the derived protein sequence and HSV as well as other thymidine kinase genes. Using similar search parameters, no homology between ORF 2 (left-most ORF, Figure 1) and HSV TK or other viral TK genes is detected nor is there detectable homology between ORF 1 and ORF 2 in the HVT sequence. The predicted amino acid sequence of ORF 2, however, does have significant homology to a predicted protein sequence found in the analogous region of HSV-2 DNA. The function of this HSV protein is unknown although mutations in this region cause reduced virus yields from HSV-1 infected cells. Based on the DNA sequence homologies and the genetic data which locate sequences imparting AraT-resistance and $^{125}$IdC incorporation to the same region, we expect that ORF 1 is the thymidine kinase gene in HVT.

EXAMPLE 2

INSERTION OF FOREIGN DNA INTO HVT USING THE "FORWARD" SELECTION PROTOCOL

The 1.3 kb kanamycin gene (Kan) of TN 903 from pUC4K (obtained from Pharmacia) was inserted into 4 restriction endonuclease cleavage sites that lie within the TK open reading frame. These plasmids were individually co-transfected into CEF with infectious wild-type HVT DNA. Cultures were passed once to expand, then cell-free virus was adsorbed to fresh CEF and AraT was added to the medium following adsorbtion. Plaques growing in the presence of AraT were purified and expanded, and viral DNA was analyzed for insertion of Kan into HVT. Insertions at the four sites, an HpaI, XbaI and two SspI sites (1-4 in Figure 1) yielded AraT resistant HVT. This demonstrates that it is possible to insert foreign DNA into this region of the HVT genome by using AraT resistance as the selection and purification mutants isolated to date have been found to grow less well than wild-type. In the case of the insertions at the HpaI, XbaI, and closest of the 2 SspI sites (1-3 in Figure 1), it is believed that the Kan insertions affect another gene in addition to TK (ORF2). It appears that the expression of ORF 2 in these insertion mutants is reduced, and that this has a deleterious effect on HVT virus production.

EXAMPLE 3

INSERTION OF FOREIGN DNA INTO HVT USING THE BACKWARDS SELECTION SCHEME

Insertion of the Kan gene from Tn903 that lie 3' of the coding region of HVT TK have been co-transfected with ATR° DNA. Because of the strong growth advantage of wild-type (WT) over AraT°, it is possible to use the accelerated growth in the recombinants as a selection tool. When the foreign DNA is linked to the rescuing DNA, it is possible to co-transduce the insertion of Kan, along with the rescue of AraT°, if the insertion of Kan does not affect the production of an adjacent, essential gene product. Six insertion sites for Kan that lie 3' of the termination codon defining the carboxy terminus of TK have been tried, 5 of these have resulted in the insertion of Kan as well as the repair of the AraT° growth defect (at sites 5-9 in Figure 1). These insertions span a one kb region of DNA that appears to be nonessential for normal HVT growth in CEF. In addition, they demonstrate that it is possible to recover HVT with foreign genes of interest using the Backwards selection strategy where HVT with a defective TK is repaired by the introduction of DNA containing wild-type TK and adjacent foreign DNA. This strategy can be extended to any nonessential site in the HVT genome by using infectious DNA from a virus carrying a defective (preferably a big deletion to avoid later recombination) TK and a plasmid carrying good HVT TK gene and a gene of interest embedded within the nonessential HVT sequences.

EXAMPLE 4

EXPRESSION OF FOREIGN DNA IN HVT

The HSV 1 TK gene carrying its own promoter (the BamHI fragment from pHSV-106, BRL) was inserted into the HpaI site (site 1 in Figure 1) within the TK gene of HVT. This plasmid was co-transfected with ATR° DNA, and plaques with wild-type growth properties were isolated. DNA from these plaques show the expected pattern for insertion of the HSV TK gene into HVT. In this case, functional TK must be made from the HSV gene.

It will be apparent to those skilled in the art that various modifications and variations can be made in the processes of the present invention. Thus, it is intended that the present invention cover these modifications and variations provided they come within the scope of the appended claims and their equivalents.

**Claims**

1. A method for introducing a gene of interest into a gallinaceous bird comprising:
   (a) inserting the gene of interest into a site in a non-essential region of a herpes virus of turkeys (HVT) to create a viral vector; wherein said site is located within the 6.25 kb Sal I fragment depicted in Fig. 1;
   (b) infecting a gallinaceous bird with the viral vector; and
   (c) causing the gene of interest to be expressed.

2. The method of claim 1 wherein said gallinaceous bird is a chicken.

3. The method of claim 1 wherein the non-essential region is located with the gene encoding TK resistance.

4. The method of claim 1 wherein the non-essential region is located at a position of the genome between two DNA sequence encoding genes.

5. The method of claim 4 wherein the non-essential region is located 3' of the gene encoding AraT resistance.

6. A method for the creation of a viral vector capable of introducing a functional gene of interest into a gallinaceous bird comprising:
   (a) locating a site within a non-essential region in the genome of a herpes virus of turkeys (HVT) wherein the non-essential site is located within the 6.25 kb Sal I fragment depicted in Fig. 1;
   (b) inserting the gene of interest into said site within the non-essential region of the viral genome to create a viral vector.

7. The method of claim 6 wherein the gene of interest is inserted into the TK region of the HVT virus.

8. A method for the creation of a viral vector capable of introducing a functional gene of interest into a gallinaceous bird comprising:
   (a) identifying a viable herpes virus of turkey (HVT) which has a genomic defect which is manifested in an identifiable manner; and
   (b) inserting the gene of interest coupled to a DNA sequence capable of repairing the viral defect into a site within the 6.25 kb Sal I fragment of the virus depicted in Fig. 1 to create a viral vector.

9. The method of claim 1, wherein said site is located 3' from nucleotide 2026 of Fig. 3.

10. The method of claim 1, wherein the gene of interest is inserted at a site selected from the group consisting of sites 5, 6, 7, 8 and 9 of Fig. 1.

11. A vector for expressing a gene of interest in a gallinaceous bird, wherein said vector is composed of a herpes virus of turkey (HVT) said gene of interest being inserted into a site within a non-essential region of said virus wherein said site is within the 6.25 kb Sal I fragment depicted in Fig. 1.

12. The vector according to claim 11, wherein said gallinaceous bird is a chicken.

13. The vector according to claim 11, wherein the non-essential region is located within the gene encoding TK resistance.

14. The vector according to claim 11, wherein the non-essential region is located at a position of the genome between two DNA sequences encoding genes.

15. The vector according to claim 14, wherein the non-essential region is located 3' of the gene encoding AraT resistance.

**Patentansprüche**

1. Verfahren zum Einbringen eines Gens von Interesse in einen hühnerartigen Vogel, umfassend:
   (a) Einfügen des Gens von Interesse in eine Stelle in einer nicht-essentiellen Region eines Herpes-Virus aus Truthahn (HVT), um einen viralen Vektor zu erzeugen; wobei diese Stelle in dem 6,25 kb Sal I Fragment, gezeigt in Fig. 1, vorhanden ist;
   (b) Infizieren eines hühnerartigen Vogels mit dem viralen Vektor; und
   (c) Veranlassen, daß das Gen von Interesse exprimiert wird.

2. Verfahren nach Anspruch 1, worin der hühnerartige Vogel ein Huhn ist.

3. Verfahren nach Anspruch 1, worin der nicht-essentielle Bereich in dem Gen, das TK-Resistenz kodiert, liegt.

4. Verfahren nach Anspruch 1, worin die nicht-essentielle Region bei einer Position des Genoms zwischen zwei DNA-Sequenzen liegt, die Gene kodieren.

5. Verfahren nach Anspruch 4, worin die nicht-essentielle Region 3' von dem Gen liegt, das AraT-Resistenz kodiert.

6. Verfahren zum Erzeugen eines viralen Vektors, der ein funktionelles Gen von Interesse in einen hühnerartigen Vogel einbringen kann, umfassend:
   (a) Lokalisieren einer Stelle innerhalb eines nicht-essentiellen Regions in dem Genom eines Herpes-Virus von Truthahn (HVT), wobei die nicht-essentielle Region in dem 6,25 kb Sal I Fragment, gezeigt in Fig. 1, liegt;
   (b) Einfügen des Gens von Interesse in diese Stelle innerhalb der nicht-essentiellen Region des viralen Genoms, um einen viralen Vektor zu erzeugen.

7. Verfahren nach Anspruch 6, worin das Gen von Interesse in die TK-Region des HVT-Virus eingefügt wird.

8. Verfahren zum Erzeugen eines viralen Vektors, der ein funktionelles Gen von Interesse in einen hühnerartigen Vogel einbringen kann, umfassend:
   (a) Identifizieren eines lebensfähigen Herpes-Virus von Truthan (HVT), das einen genomischen Defekt besitzt, der in einer identifizierbaren Weise manifestiert ist; und
   (b) Einfügen des Gens von Interesse, gekoppelt an eine Sequenz, die den viralen Defekt reparieren kann, in eine Stelle in dem 6,25 kb Sal I Fragment des Virus, gezeigt in Fig. 1, um einen viralen Vektor zu erzeugen.

9. Verfahren nach Anspruch 1, worin die Stelle 3' von Nukleotid 2026 aus Fig. 3 gelegen ist.

10. Verfahren nach Anspruch 1, worin das Gen von Interesse in eine Stelle eingefügt wird, ausgewählt aus der Gruppe, bestehend aus Stellen 5, 6, 7, 8 und 9 aus Fig. 1.

11. Vektor zum Exprimieren eines Gens von Interesse in einem hühnerartigen Vogel, wobei sich der Vektor zusammensetzt aus einem Herpes-Virus aus Truthahn (HVT), dem Gen von Interesse, das in eine Stelle in einer nicht-essentiellen Region des Virus eingefügt ist, wobei diese Stelle in dem 6,25 kb Sal I Fragment, gezeigt in Fig. 1, liegt.

12. Vektor nach Anspruch 11, worin der hühnerartige Vogel ein Huhn ist.

13. Vektor nach Anspruch 11, worin die nicht-essentielle Region in dem Gen, das TK-Resistenz kodiert, gelegen ist.

14. Vektor nach Anspruch 11, worin die nicht-essentielle Region an einer Position in dem Genom zwischen zwei DNA-Sequenzen gelegen ist, die Gene kodieren.

15. Vektor nach Anspruch 14, worin die nicht-essentielle Region 3' von dem Gen, das AraT-Resistenz kodiert, gelegen ist.

**Revendications**

1. Procédé d'introduction d'un gène d'intérêt dans un gallinacé comprenant les étapes consistant à :
   (a) insérer le gène d'intérêt dans un site d'une région non essentielle d'un herpèsvirus de dinde (HVT) pour créer un vecteur viral; dans lequel ledit site est situé dans le fragment Sal I de 6,25 kb présenté dans la figure 1;
   (b) infecter un gallinacé avec le vecteur viral ; et
   (c) provoquer l'expression du gène d'intérêt.

2. Procédé de la revendication 1, dans lequel ledit gallinacé est un poulet.

3. Procédé de la revendication 1, dans lequel la région non essentielle est localisée avec le gène codant pour la résistance TK.

4. Procédé de la revendication 1, dans lequel la région non essentielle est localisée en une position du génome située entre deux séquences d'ADN codant pour des gènes.

5. Procédé de la revendication 4, dans lequel la région non essentielle est localisce en 3' du gène codant pour la résistance à l'AraT.

6. Procédé pour la création d'un vecteur viral capable d'introduire un gène d'intérêt fonctionnel dans un gallinacé comprenant:
(a) la localisation d'un site à l'intérieur d'une région non essentielle dans le génome d'un herpèsvirus de dinde (HVT) dans lequel le site non essentiel est situé dans le fragment SalI de 6,25 kb présenté dans la fig. 1 ;
(b) l'insertion du gène d'intérêt dans ledit site à l'intérieur de la région non essentielle du génome viral pour créer un vecteur viral.

7. Procédé de la revendication 6 dans lequel le gène d'intérêt est inséré dans la région TK du virus HVT.

8. Procédé pour la création d'un vecteur viral capable d'introduire un gène fonctionnel d'intérêt dans un gallinacé comprenant :
(a) l'identification d'un herpèsvirus viable de dinde (HVT) ayant un défaut génomique qui se manifeste de manière identifiable ; et
(b) l'insertion du gène d'intérêt couplé à une séquence d'ADN capable de réparer le défaut du virus dans un site compris dans le fragment SalI de 6,25 kb du virus présenté dans la fig. I pour créer un vecteur viral.

9. Procédé selon la revendication 1, dans lequel ledit site est situé en 3' du nucléotide 2026 de la fig. 3.

10. Procédé selon la revendication 1, dans lequel le gène d'intérêt est inséré en un site choisi dans le groupe constitué des sites 5, 6, 7, 8 et 9 de la fig. 1.

11. Vecteur d'expression d'un gène d'intérêt dans un gallinacé, dans lequel ledit vecteur est composé d'un herpèsvirus de dinde (HVT), ledit gène d'intérêt étant inséré dans un site compris dans une région non essentielle dudit virus, dans lequel ledit site est compris dans le fragment SalI de 6,25 kb présenté dans la fig. 1.

12. Vecteur selon la revendication 11, dans lequel ledit gallinacé est un poulet.

13. Vecteur selon la revendication 11, dans lequel la région non essentielle est localisée dans le gène codant pour la résistance TK.

14. Vecteur selon la revendication 11, dans lequel la région non essentielle est localisée en une position du génome située entre deux séquences d'ADN codant pour des gènes.

15. Vecteur selon la revendication 14, dans lequel la région non essentielle est localisée en 3' du gène codant pour la résistance à l'AraT.

FIG. 1

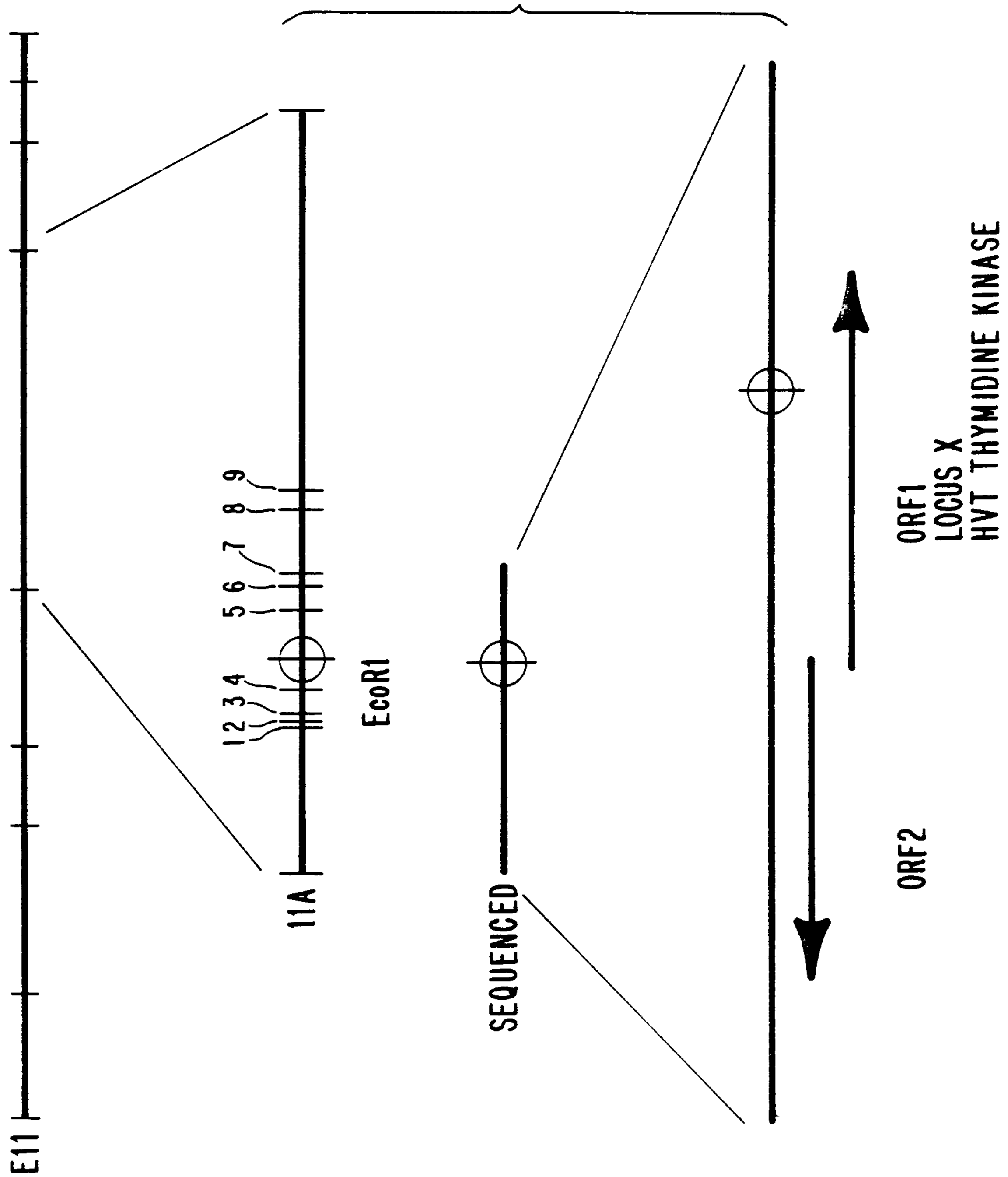
E11
11A
1 2 3 4 5 6 7 8 9
EcoR1
SEQUENCED
ORF2
ORF1
LOCUS X
HVT THYMIDINE KINASE

FIG. 2

MARKER RESCUE OF ATR°

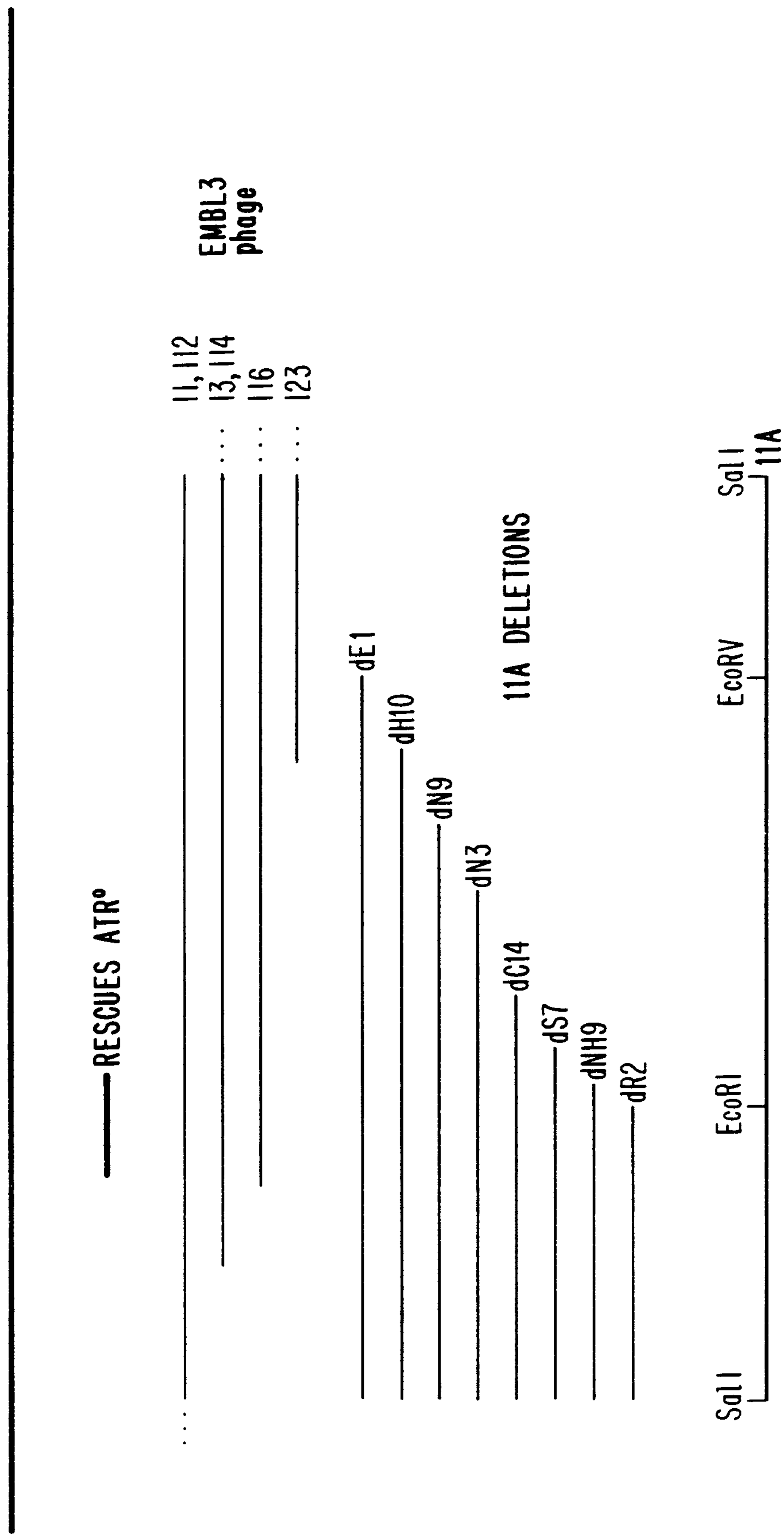

# FIG. 3

```
   1 TCGACGCCAT CTCTTTCAAT TTATCCTCCA AAGGTCTGAG TCTGCGGTCA ACTTCCACCG
  61 CAATAGTATT GGTGCGACCA TCTAAATCGT CTAATGCTGC AGCGGCAGCC TTGTTGCGTG
 121 CCGTCACGAG GGCGAGTTGT TTTTCCAATG CCCCACACTC ATCGGTAAAA GCGGGGTCTT
 181 TCCAAAACTG TATTGGCCAA CGGGGGGCAA TAAAATTCTT CGTATCGGCC GGGTGTGATT
 241 CCAAGTGGTC ATTTCCCATA ATCCTGGCCC CCCAAATGAA ATTGACCATG TCTCTAGACA
 301 TTTTAAGGAC GTGTTAATGT GCCGGGGCCA CTATATTCCT AGCAGCCGCA CGTCGCGTAT
 361 TTAAATGTGA AGGTCAACCT AACAGGCGGT GTCTACATTA CACCCGCGAT AAACTTTGCA
 421 GACTGCCCCT TTTTAAATCG TTAATGCCTT CGCGATAAGT GCGATATGAT GTTACGCATG
 481 CGCTGCATTA TCGGTTCTCC TTGAATGCCA GATGTTCCAC AGTCCTTACC GCGTGCATCT
 541 ACATGCTGTT TTTCGATTGG AAGCACACCA GTATCAGAAA TCCCACATCG CATTCTACGT
 601 ACAGTGCTCA CCGTTCGTTT ATTACGATGG CAAATTTTAC GCATGGGCTT GCACATACCC
 661 GAATCATAAA GCCTATATTC TGATCTGAGA TACATCATTG ATGCCAATCG ATGAAAGTCT
 721 GTACATATAG CCTTCTTTTT TAATGGTGTC GAACGTATAA CTACAAGCCC GCGTCTCGCG
 781 ACAAACACTA AAACGGGGCA AATGATAGTA TGATCAGATC CCCTGGGGGC CATGCGCTCG
 841 ATCAACGTTT TAGACTCCAG CAACTGCTTA GTCCCTGTTG CGCGTTGCTC ATTCTTGCTG
 901 GCCGTCTTGA GGTTGCGGTA AAATCTGCAA GTTTTCAGTT CGATTATGAC ACAGACATTG
 961 TTACAGCCTT CCACCAATAA CCCGCCAGAT AAGGTGAACA TGCAAATGCA ATCTGGCCTT
1021 CTGCGCCCCA GGTCTACTTC AAAGGCCAAT CGAAACGCCG ACGCCCCTTT AAGTAGTTCA
1081 GAAGGCAGGA TCTTGCTTAA TATCTTCTGT AATGTTCTAC CAACTGTATT CCCCGCCGAC
1141 GACTGGTCAA AGTTTTCAGC AGCAAGCTTT TTGTAAAAAC GATTATGACC ACGGACACCC
1201 GCTTTTAGCA ATCCTGCCAT AAGGTGGTTT CGCCGCGTGC TTGCCTCGAA GACAATTGCC
1261 AGCTAATCCA GCATTACCAT ATTTCCTTGG CTTGCATTTG GATCTGCGCG TCGATGGCAT
1321 TGCCGAGAAG ACCGCCCACG TTAACGCGAG TTTATCTAGA CGGACCGTTT GGTATAGGCA
1381 AAACGTCTAT ACTAAACGCT ATGCCCGACC ACACGCCCGA TGGGGCTCCT ATATTGAAAG
1441 TGTACGAACC AATGAAATAT TGGAGATGCC AGTCTACCGA TTTGGTGGTA GCTGCCAACG
1501 AAACGCCAGA ACGTAGGCGT GGTGGAGCTT TATCACGATT CCAATCTGAC ATGATCATGG
1561 CATCTATACA AGCCAGATTT GCCGATCCAT ATTTGCTTTT TCACGAACGG TTATCATCTA
1621 AATGTAGAGG AAAAATAGAA ATATGCGATA CTCCAGCAAT TATATTAATG CTGGATAGGC
1681 ACCCTGTGGC GGCGATATTA TGTTTCCCAA TCACTCGCTA TTTACTTGGA GAATATTCTT
1741 TGGAAATGTT GATTAGCTCT ATAATAAGAC TTCCGTTGGA ATCCCCCGGA TGCAACCTGA
1801 CAGTCACAAT CCTTCCCGAC GAAAAGGAAC ACGTTAATAG GATTTGTTCA AGAGATAGAC
1861 CGGGTGAAAC GGCAGATAGA AATATGCTCA GAACACTCAA TGCCGTATAC GCATCTTTGG
1921 TGGACACGGT TAAATACGCA AATCTAACAT GCCCTTACGA GAAAGAAAGC TGGGAAATGG
1981 AATGGTTGGG ACTTCCCTGG TTTGAAGAGT CATTACTTGA AGAATTCATC TCGCGTCCCC
2041 GCCCTGTTAT TTGTTCGAGA ACTCGAATGC CGCTGGACCG AACTCTCCTG GCCATTTTTA
2101 AACGGAAAGA GCTGTGTAGC GAAAATGGGG AGCTGTTAAC TCAGTATTCT TGGATATTGT
2161 GGGGATTACT GACTAAACTA CACACCATTA ATGTCGAATT ATTTGACATT AGCGGTATGT
2221 CACGTCGAGA ATGCGCCACG CTATAATGCA TACTATGCCG GAGAGATTGT CTACTCTCGC
2281 TAGCTGGAAT GATTTATGCG AGCTTGAAGA TGATGTAATT TCCTATAATA AGGGAATGTG
2341 TAACGAGGTT GGAGCGTCTC GATAATTCTT CTTAATCTGC TGGTATTGGT TACTGCCATA
2401 ACTTAATATT GGTCCATGCT AGAATAGTCA TACGCTACGA TCTGTTGCTA TATATGACTA
2461 TCGCCAAACT GTTAAACCGC GAAGAATATA TTTCATATAA ACCTAAGGGC CCCTCAGTCT
2521 GATTTTTTGT GAAAACGTGT ATACATGAAG TTTTACTGCA TAATCCGTTT CATGATCATA
2581 GCGAATCTTT ATTCATCTTA CCAAATATCG CTTCCAGGCA CATATCCATC GCAAATATTG
2641 CTTGACATGA AGAACTCGCC GCTCGTACGC TTTAATATAT CGACGCGTGA TTATAAAGAC
2701 GAGACACTCT GGATACGGAA
```